# EUROPEAN PATENT APPLICATION

(11) **EP 4 685 772 A1**
(43) Date of publication of application: **28.01.2026**
(21) Application number: 25189657.7
(22) Date of filing: 15.07.2025
(51) Int. Cl.: G09B 19/00, A61B 5/00, G06F 3/0346, G16H 20/30, A61B 5/11, G16H 40/63, G16H 40/67, G16H 50/20, G16H 50/70

(54) **IMPROVED METHOD AND APPARATUS FOR SPORTS AND MUSCLE MEMORY TRAINING AND TRACKING**

(30) Priority: 23.07.2024 TW 113127548
(71) Applicant: HSUH TA ENTERPRISE CO., LTD., New Taipei City 238027 (TW); Liu, Wei-Yu, New Taipei City 238027 (TW)
(72) Inventor: LIU, WEI-YU, 238027 New Taipei City (TW)
(74) Representative: Santarelli

(57) **Abstract**

An accelerometer is mounted on a location of a user's body, sports equipment, or other device and tracks movement. Data describing the user's movement(s) may be saved or uploaded for distribution. The user's movement(s) may be compared to previously saved or downloaded movement data and an audio, vibrational, electrical, or other alert may be activated when the movement data is outside a predetermined range from the previously saved, pre-programmed, or downloaded movement. The invention is useful at least in various forms of physical therapy, sports training, and job training, and provides synergy when utilized in conjunction with other motion help related devices.

## Description

### Cross Reference to Related Applications

The present application claims the benefit of Taiwanese Patent Application No. 113127548 filed on July 23, 2024, the contents of which are incorporated herein by reference in their entirety.

### Copyright Notice

A portion of the disclosure of this patent document contains material which is subject to copyright protection. The copyright owner has no objection to the facsimile reproduction by anyone of the patent document or the patent disclosure, as it appears in the Patent and Trademark Office patent file or records, but otherwise reserves all copyright rights whatsoever.

### Background of the Invention

### Field of Invention

The present invention relates to training and physical therapy and particularly to movement and training muscles to follow exemplary paths such as those prescribed by a physician/therapist, a desired path of motion in a sporting activity, or other uses where tracking and/or feedback or muscle or other motion can be used advantageously.

### Description of Related Art

Currently many devices are available for viewing and commenting on motions. For example, video is commonly used in physical therapy to instruct patients how to perform particular motions having benefit for the patient's recovery. Sports players often review tapes or video to analyze and improve motion such as their form when kicking a football or pitching in baseball.

### Summary of the Invention

The present inventor has realized the need for improved tracking of motion and feedback to a patient or sports player. In one embodiment, the present invention provides a wearable device that detects motion data and provides feedback according to a comparison or other analysis.

The present invention realizes that simplicity in operation is a valuable commodity, and, in an efficient form, the invention comprises an electronic device with accelerometers that detect motion and provide direct and immediate feedback to a user. Such feedback is, for example, through audio (e.g., high/low left/right, etc.). The Muscle Memory Device (MMD) is preferably arranged so as to be pocket sized or watch sized (or smaller), and is a device that can record its motion and then use the recorded motion to provide feedback to a user. The MMD uses a gyroscope to measure angular velocity about 3 axes: x, y, z. With this, the object's orientation (equipment, user's hand, foot, etc.) in 3D space. An accelerometer measures acceleration (a change of velocity) across single axis. Multiple accelerometers may be utilized. A magnetometer may be used to detect the Earth's magnetic field, to determine motion, orientation and heading. In conjunction with the accelerometer(s) and gyroscope, an absolute heading of the device may be determined.

In one embodiment, a power button initiates the device, powering it up ready. A selector switch is set to a position for recording, and the MMD is ready to record the user's motion, saving it in memory. Upon setting the switch to a play position, the device will stop recording user's movement and is ready to analyze the user's motion compared to the recording.

The invention includes removing irrelevant motion data before the start of the move and after the move, but in some embodiments, keeping enough data before the motion if it is relevant for use in embodiments that pre-identify a motion the user may be about to perform.

In analyze mode, the user performs a motion. If the results match (the user's motion matches the recording), the device produces, for example, a distinguishable beeping sound (or a chime, or no sound at all). If the results don't match, the device produces different beeping sounds. Non matching sounds or alerts/alarms may be more pressing in nature, encouraging the user to do something different. In one embodiment, the sounds are coaching voices, such as instructions to "pull up," "lower," etc. The sounds are produced in real time, so during, or at least right after each movement execution, the user gets instant feedback to know whether the move execution was successful or not.

With wireless capability (e.g., Bluetooth), the MMD can be connected to a smartphone, server, the Internet, or other devices (including other MMDs). When connected to a smartphone, data recorded by the MMD (e.g., all data or move/motion specific data) can be stored in the phone. Individual moves or motions may be stored on the smart phone and may be uploaded to the MMD's memory. The smart phone may provide a platform for an application to facilitate the above, select moves and/or motions, and to identify tweaks that may be desired to the various moves (e.g., faster, slower, varying force, etc.).

With the use of the app, the users can store multiple recorded movements and select among the recorded movements and/or downloaded movements.

With the flicker (switch) in its right (or play) position, the users can then decide which movement they want to practice (recreate). With the flicker in its left (or record) position, the users can record more movements. The smartphone connection is optional and not necessary for the use of the device. The user may select from multiple stored movements without a smartphone app by, for example, moving the selector switch back and forth 3 times to select the 3rd recorded movement. In one embodiment, an additional switch with multiple positions is added to select the potentially multiple recorded movements.

In yet another embodiment, the functions of the switch selector 401 and power button are combined into a single button. For example, the MMD can be powered-up by pressing the button, and powered-down by pressing the button for a predetermined length of time (e.g., 3-seconds or longer). Changing between modes may be performed by holding the button down for another predetermined length of time (e.g., 2-seconds to change modes - e.g., switch between record and play modes). If the MMD holds multiple programs, the programs may be cycled by yet another duration press (e.g., a momentary press changes programs - e.g., momentary press switches between recorded program 1 (forehand), recorded program 2 (backhand), and possibly others). In one embodiment, the MMD signals the program change by a beep or series of beeps (e.g., 1-beep for program 1, 2 for program 2, etc.).

Pre-recorded moves (e.g., from professional table tennis players, tennis players, and other movements that require professional players to do perform them) can be downloaded with the use of the app. Programming and physical and/or wireless connections to the MMD may also provide a direct way to download movements that may be, for example, selected using a website and then automatically downloaded to the MMD.

The MMD can record statistics of a user's movements, and users can upload their statistics and their own movements online, or share them via social media. With all the data stored for any given movement, the move can be displayed on the smartphone's screen visually so that the user can evaluate/visualize the move, see how the MMD was moving in space, etc.

The MMD is not only useful for specific sports. It can be used for any desired movement - sport, muscle training, physical therapy, etc. In one embodiment, a physical therapist, doctor, or coach can directly upload motions into a patient or athlete's MMD, and a professional's app or computer program can maintain a set of preferred or prescriptible (prescribed motion) motions and allow the professional to enter an alert, notification, text, or email with any explanation or notes the professional may want to convey to the user. The present invention includes automatically entering a billing line into a patient or athlete's account for uploading the motion and any review, discussion, or analysis performed by the professional.

It can aid for training of table tennis serves, kicking soccer ball in a specific user determined way, aid in walking in a specific user determined way, help with various user determined muscle movement training. All movements are performed by the user and the device does not claim to help with healing injuries, nor is it responsible for improper use. However, if certain motions are helpful in the healing process, such as those prescribed by a physician or physical therapist, the motions will have benefit.

The user may determine exactly what kind of movement, he wishes to execute, and the device aids the user to perform the specific move into perfection by repeatedly practicing it. Just like when we walk, we don't intuitively think "I'm going to lift my right leg up, then move it forward, then put it down and repeat the same with my left leg". We do the moves subconsciously, where our muscles are trained to do moves without the "how to do them thinking". Repeated use of the MMD helps move that process along for any recorded motion.

In different embodiments, the MMD can help with performing very specific moves in a way that helps with muscle regeneration after injuries. Various movements are done for physical therapy. If recorded correctly, then the user can use the MMD to execute those movements with instantaneous feedback. This can serve as an additional help with physical therapy (or muscle strengthening - to avoid medicinal use).

In another embodiment, the present invention provides a method of analyzing motion comparted to a desire range of motion. The present invention includes an application on a phone, computer, or other electronic device that receives motion data, compares it to a baseline, and sends feedback signals in real or near real-time such that a user knows immediately if the motion is correct or off-track. The feedback may include an audible alarm, vibration, electrical signal, etc.

The present invention includes multiple types and instances of feedback for the adjustment or correction of motion. For example, in one embodiment the present invention retrieves stored and/or real-time data and displays caricatures or images on a smartphone performing a motion performed by a user along with indicia of differences between the motion and a desired motion. The differences indicia include, for example, highlighted body parts or portions of body parts outside acceptable limits of the motion as the caricature or image performs the motion (e.g., playback of the user's motion with indicia added).

The indicia may include motion streaks, arrows, that help suggest the desired motion. Such streaks or arrows may, for example, be placed at critical junctures before the incorrect motion occurs which preconditions the reviewing user before viewing the highlighted incorrect motions. Advantageously, the differences indicia may correspond directly to audio or vibrational feedback the user received while performing the motion. Such review provides double cues that reinforce the proper motion. In yet another embodiment, the vibrational or audio feedback is repeated while reviewing a caricature or image playback (with or without visual indicia of errors or correct motion) so as to provide yet another correlation to the proper motion and adjustment required to perform it properly.

In one embodiment, the feedback is primarily audio having a sound that is related to the motion being performed. The present invention includes methods for identifying moves and comparing motion to motions stored in a database, and others.

Portions of both the devices and methods may be conveniently implemented in programming on a general purpose computer, or networked computers, and the results may be displayed on an output device connected to any of the general purpose, networked computers, or transmitted to a remote device for output or display. In addition, any components of the present invention represented in a computer program, data sequences, and/or control signals may be embodied as an electronic signal broadcast (or transmitted) at any frequency in any medium including, but not limited to, wireless broadcasts, and transmissions over copper wire(s), fiber optic cable(s), and co-ax cable(s), etc.

### Brief Description of the Drawings

A more complete appreciation of the invention and many of the attendant advantages thereof will be readily obtained as the same becomes better understood by reference to the following detailed description when considered in connection with the accompanying drawings, wherein:
Fig. 1 is drawing illustrating a band having an attached Muscle Memory Device (MMD) according to an embodiment of the present invention.
Fig. 2 is a block diagram illustrating components and communications according to an embodiment of the present invention;
Fig. 3 is a diagram illustrating software and programming according to an embodiment of the present invention;
Fig. 4 is a photograph of prototype electronics configured to implement an MMD according to an embodiment of the present invention;
Fig. 5 is an illustration of a sports device having an attached MMD according to an embodiment of the present invention;
Fig. 6 is a drawing of an MMD attachment mechanism according to an embodiment of the present invention;
Fig. 7 is a flow chart of a motion comparison process according to an embodiment of the present invention;
Fig. 8 is a screen shot of an application according to an embodiment of the present invention;
Fig. 9 is a diagram of a MMD according to an embodiment of the present invention;
Fig. 10 is a diagram of internal components of a MMD according to an embodiment of the present invention; and
Fig. 11 is a diagram of internal components of a MMD according to an embodiment of the present invention.

### Detailed Description of Embodiments

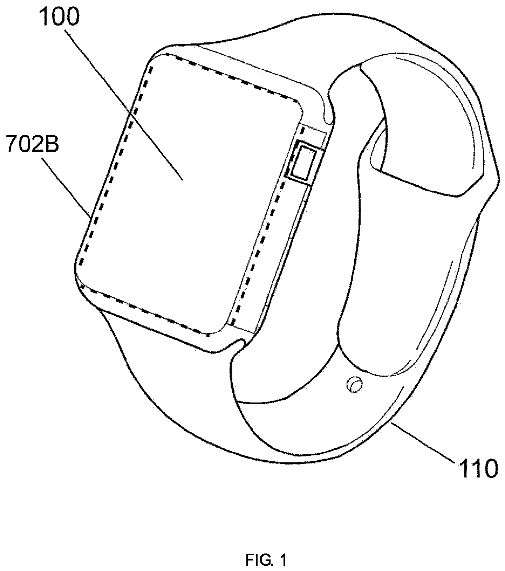

Referring now to the drawings, wherein like reference numerals designate identical or corresponding parts, and more particularly to Fig. 1 thereof, there is illustrated a Muscle Memory Device (MMD) 100 according to an embodiment of the present invention. The tracking sensor is attached to a band 110 which may be affixed to a wrist, foot, or other appendage of a patient or sports trainee. In another embodiment, the MMD 100 may include adhesive for attachment directly to the skin at various locations of the user.

The MMD 100 may be configured to operate in different ways. In one embodiment, the MMD 100 operates as a motion tracking and alert system. For example, the MMD 100 is programmed to recognize physical therapy movements and alert the user at predetermined points in the movement. Such alerts occur, for example, when the user moves outside a range of motion deemed appropriate for the therapy maneuver being performed. The alerts are, for example, real-time alerts notifying the user the moment the user's range of motion deviates sufficiently to be incorrect.

For example, a user is prescribed a movement such as a rotating leg lift (e.g., lifting leg with a small amount of clockwise then counter-clockwise rotation). If the user fails to lift the leg high enough, an alert or alarm sounds. Similarly, if the user fails to rotate along with or during the lift, an alert or alarm sounds. As will be discussed in more detail further below, the same type of alert or alarm may be implemented in other areas, such as sport moves, part of sporting equipment, practice in professional moves in many different fields, etc. The alert or alarm may be initiated based on other than improper moves, such as a reward for extra extension other than that prescribed if deemed appropriate (e.g., additional extension in some cases might not be desirable). Such alerts and/or other feedback may be provided via lighting such as LEDs mounted on or under a surface of the MMD, such as lighting feedback 702B, and have capabilities as further described hereinbelow.

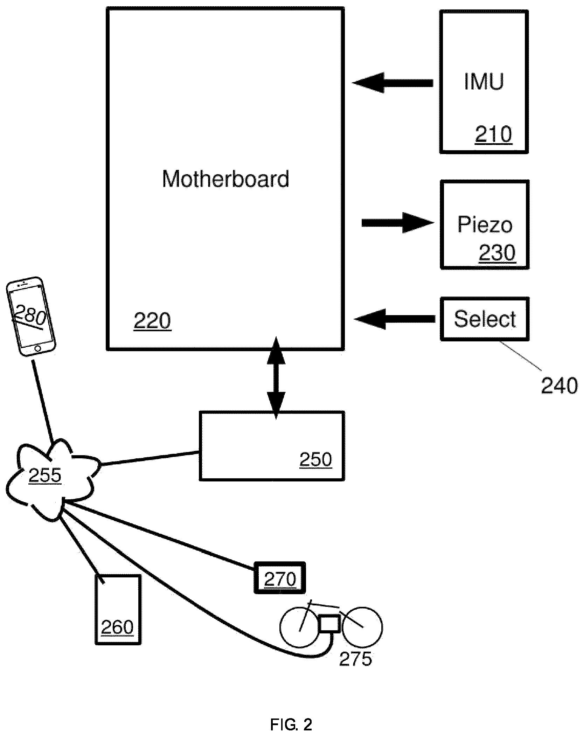

Fig. 2 is a block diagram 200 illustrating components and communications according to an embodiment of the present invention. An IMU 210, which, for example, is embedded in the MMD 100, senses motion in various axes, and provides the motion data to a motherboard having a central processing unit and programming to implement the present invention. A compass, GPS, or other device for direction finding may also be included. The programming may include, for example, data storage of inertial measurements, averages, or samples from one or more of a variety of physical therapy or sports motions (actual moves or training exercises, for example) and routines that compare a user's motion to a stored motion.

For example, in one embodiment a stored motion may be stored based on position, velocity, and acceleration over a predetermined number of samples per second. A pattern of samples is matched with real-time data from the IMU 210, where the comparison is immediate and a decision is made whether the user's current motion is correct or deviating from a corresponding sample or samples. When deviating the alert/alarm is sounded (e.g., piezo 230), notifying the user to correct or improve the motion. Upon sufficient correction, the alert/alarm is silenced.

In one embodiment, the alarm is customized based on the motion. For example, the alarm may give a cue as to the appropriate motion or corrective action needed. In the case of an audio alarm, if the user's motion is from a high position to a low position, and the stored movement is from a low position to a high position (or if just generally, the user's motion needs to be more up than down) the alarm may be a tone that varies from low to high - an audio cue as to how the motion should proceed.

The MMD 100 may include a select switch 240 that inverts moves by switching between a left-handed (or left-sided) motion to a right-handed (or right-sided) motion. Changing from a left to right-sided (or visa-versa) motion may be performed via a mathematical translation of the stored move from a first-side perspective to a second-side perspective. In one embodiment stored moves may be adjusted for an individual user's particular circumstances. For example, in the case of a patient or trainee with a prosthetic limb, the stored data may account for non- standard movements made because of the prosthetic. Adjustments may also be made to account for varying user sizes (taller, shorter longer, heavier, etc.) Accordingly, the present invention includes a mechanism to adjust moves (e.g., professional moves downloaded from the Internet) such that they fit physical characteristics of an individual user.

More than one motion or move may be stored in the MMD, and a select switch 240 may be configured to select different moves or measurements from different positions (e.g., wrist or ankle). The select switch 240 may also be used to set the MMD into record mode, where a physical therapist, instructor, or trainer record a motion for the user to perform.

Fig. 2 further illustrates communication between the central processing of the MMD and an I/O device 250 which may be configured to communicate with a network or specific devices such as a cell phone 280, a robot or other device 260, a peloton-like workout machine 275 or it's corresponding visual display 270. In the illustrated embodiment, communications connect to the cloud for data storage, processing, or other tasks, and/or communicate through the cloud to mobile device 280 (where further processing or selections may occur via an application, such as those described elsewhere herein), to robot 260 (e.g., a ping pong robot receiving instructions or data from which shot decisions may be determined), to the mechanics, coaching, or workout display (e.g., terrain displayed on 275) of a peloton-like device. Such communication may include instructions that change dialog from a virtual coach associated with any of the devices, or data upon which the virtual coach programming may determine dialog (which itself may be determined "in-the-cloud" and transmitted to the device).

Such communications into the cloud may also feed other devices or platforms, such as Roblox, or an app, where, for example, a user's progress or consistency in using the MMD device may be credited with points or other indicia that may later be used in that app or platform (e.g., a user's progress on a Peloton-like device may be translated into more points or a level-up in a bicycle related game on, for example, the Roblox platform). The device may further be linked directly to the platform or game on the platform or an app such that the game or display of the game is adjusted in real-time relative to the user's performance in matching a motion, for example. Although illustrated as a communication via the cloud, the same may be performed via direct communications over any network or directly to the device.

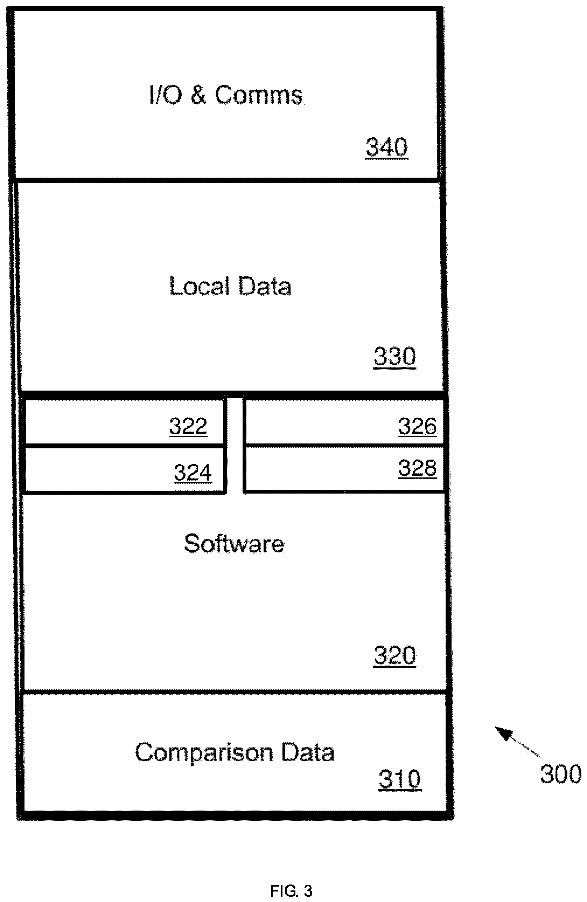

Fig. 3 is a diagram illustrating software and programming 300 according to an embodiment of the present invention. Comparison data 310 (e.g., stored, recorded, or downloaded motions/moves). The Comparison data may be recorded using the MMD 100 or another device. The comparison data may be downloaded from the Internet from a doctor, therapist, or sports enthusiast website.

Software 320 which may be, for example, downloaded software in executable form, firmware, or other electronics or programming types. The software 320 reviews IMU data and thereby compares a user's motions to a pre-recorded motion, and sets alarms as appropriate. The software 320 may optionally perform additional functions as described elsewhere herein. Additional functions that may be included, such as determining a portion of a motion 322, varying feedback relative to tolerances during a selected portion of a motion 324, communicating with an app for making selections 326, and other functions described elsewhere herein 328, for example.

Local data 330 may include, for example, buffered storage of IMU data, sections data, etc., and I/O & Comms 340 may include programming or data from any of data to or from the other components, and/or information protocols for wireless communications (e.g., Bluetooth) that may be utilized in other embodiments, expansion functionality, and retrieval of additional data or programming such as downloaded motions or moves, updates, etc. Other local data may include tolerances for comparisons of a stored motion to different portions of a motion, preferences for feedback or combinations of feedback, and/or how the feedback is presented, for example as further described herein.

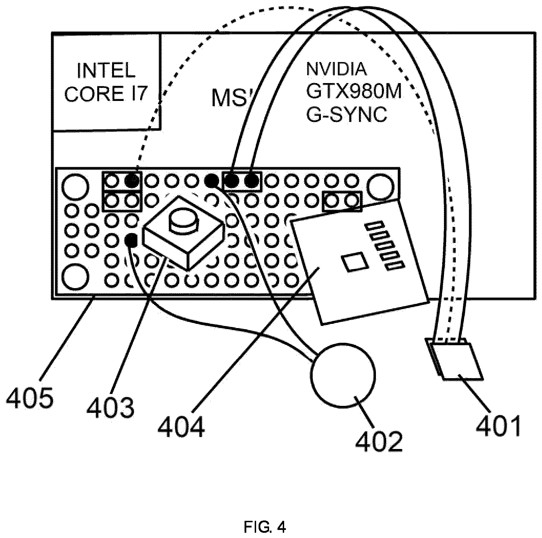

Fig. 4 is a photograph of prototype electronics configured to implement an MMD 400 according to an embodiment of the present invention. As illustrated in Fig. 4, the invention may be implemented in an architecture including a motherboard 405 (e.g., Blue fruit feather board), IMU 404, power switch 403, piezo 402, and switch/selector 401. The MMD 400 illustrated is a prototype that implements the present invention by running software 320 on the motherboard and communicating with the IMU 404 and piezo 402.

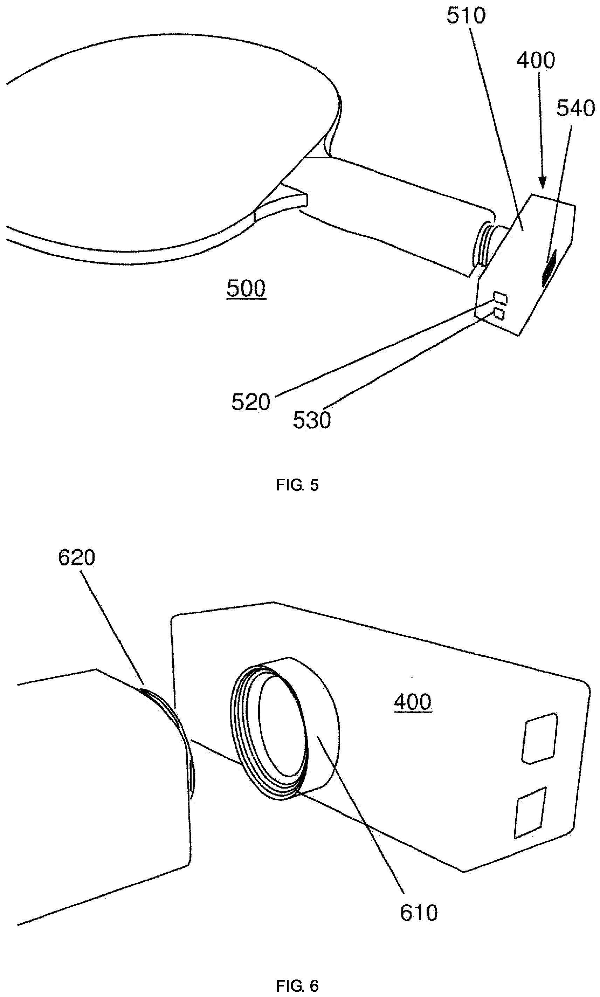

Fig. 5 is an illustration of a sports device 500 having an attached MMD 400 according to an embodiment of the present invention. Packaging 510 surrounds and encloses the protype MMD (MMD 400) such that the hardware is enclosed (so no electronics are sticking out). In one embodiment, it is desirable to have only a minimal amount of controls accessible on the MMD, which can be none (non-replaceable battery - or self-generating power supply, auto on/off, and Bluetooth downloadable motions, as one example). In the illustrated embodiment, only the power button and the flicker switch (e.g., switch/selector switch 401) are accessible and are placed so the user can interact with them. Preferably their functions are limited so the design is clean and easy to operate. Nonetheless, additional functionality may be added to the existing switches (e.g., pressing buttons twice to change programs, Multi-position selector switch, etc.) and included in other embodiments.

The packaging 510 is attached, for example, to sports equipment, and places the MMD at a location that allows the MMD to perform its functions by recording and/or comparing user movements for various strokes such as serves and returns in their numerous different forms.

User accessible power button 520, selector switch 530, and piezo (speaker) port 540 are also shown in Fig. 5. A power port for charging batteries (not shown) may also be included ([in various alternatives other power mechanisms may be utilized such as a solar panel (e.g., along a handle of a device), or an internal power generation mechanism such as one based on motion which can gain synergy from activities with significant amounts of motion.]).

Fig. 6 is a drawing of an MMD attachment mechanism 600 according to an embodiment of the present invention. As illustrated the attachment mechanism comprises a threaded mount 620 attached to the sports device and a correspondingly threaded receiver 610 attached to packaging 510 of the MMD 400.

The mounting may be attached to any number of surfaces (or internal to a piece of equipment). The MMD itself may be attached any number of different mounts and mounting locations, including a small mount that goes underneath a table tennis paddle, a small mount that goes underneath a tennis racket, a wrist band to which the MMD can be attached (e.g., band 110 in Fig. 1 as one example).

The mount may include a shoe mount which is attached to the shoe (e.g., two bands on the side of the shoe). The MMD is then attached to the mount. The MMD may also be glue mounted onto a shoe or other equipment. Preferably, in a retail package for example, the MMD includes mounts for one or more surfaces, such as a flat surface, round surfaces, and mounts that can either be changed or adapt to different surfaces (e.g., flexible or cushioned mounts that conform to an irregular surface, for example. Each mount fitting to the MMD. Various other attachment mechanisms including adhesive, individual screw(s), latches, hook & loop (e.g., Velcro TM), etc., may be utilized.

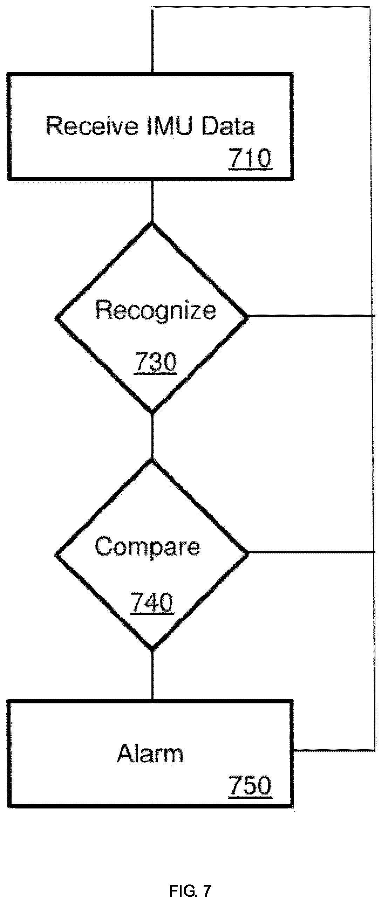

Fig. 7 is a flow chart of a motion comparison process 700 according to an embodiment of the present invention. At step 710 IMU data is read. The IMU may be a multi-axis IMU, and may provide a position in 3D space, acceleration, and velocity. Other data may also be provided by the IMU depending on the board selected for implementing the MMD. Further additional features (and corresponding data) may be provided by add-on hardware or devices (e.g., such as altitude, lat/long, data from other equipment, other MMDs on a same user or another user, etc.).

In one embodiment, the present invention uses wireless communications to transact coordinated motions such as those between a quarterback and a receiver. In such embodiments, a 5G chip (not shown) may be fitted or connected to the motherboard to transmit instantaneous data to a remote server that coordinates timing, responses, in such synchronized activities and for individual activities as well.

Decision box 730 determines if the data being provided by the IMU is a selected or stored motion. The decision may need additional or previous cycles of IMU data which may be stored in local memory such as RAM. In one embodiment, a current motion is identified piece meal such that each cycle of IMU data is determined to be either consistent or inconsistent with a selected or stored move with respect to a number of previous IMU cycles (note consistent with a selected or stored motion is not necessarily the same as an acceptably correct motion (it may or may not be acceptable)). If the motion/move is not identified, the program loops back for the next IMU cycle. The recognition may be, for example, recognition of a specific portion of a motion that user wishes to work on.

Once enough IMU cycles have been received to identify the move, the compare is activated at step 740. Step 740 may be implemented in any manner that determines if the IMU data received matches or is within a predetermined tolerance of a stored move or motion. This may include, for example, a direct comparison of IMU sample values vs. a database of moves each having a similar set of samples. Each motion may be broken down into segments, such as natural segments of a swing (e.g., backswing, approach, impact, and follow-through), and specialized comparison routines invoked for each segment. In one embodiment, the saved or recorded motions are fitted to a curve and each corresponding IMU sample is compared to a corresponding location on the curve +/- a tolerance. Regardless of the method implemented, a pass or fail is determined for a current segment of the motion and, if failing, the alarm system is activated (step 750). If pass, the program loops back for the next IMU sample.

In one embodiment, a more detailed analysis of the collected IMU data is performed to determine how much or in what direction the user's motion is failing. If failing to one direction a certain tone or frequency of alarm is produced and, for example, either increasing or decreasing in volume or frequency to indicate whether the user's motion is getting worse or better. The comparison may be, for example, comparison of a selected portion of a motion.

In yet another embodiment, the Alarm takes the form of a varying tone relative to an amount of deviation from a desired motion, and the tone, for example, is only provided during a specific identified portion of the motion. A separate tone may be provided before prior to the specific portion of the motion for continuity if desired and may be set in user preferences.

In one embodiment, the MMD stores a plan for several different moves to be performed at different locations, such as a fitness track having stations. The location of the user at the fitness track triggers the MMD to evaluate the specific motion to be performed at that station (e.g., the users location selects the motion or a subset of saved motions for comparison).

As noted above, some embodiments of the invention include 5G communications, which can be arranged in many ways. In one embodiment 5G communication may be directly from the MMD to a remote server over a 5G network. All functions of the MMD and/or previously connected smartphone may be performed at the remote server and signaling for the application of haptics, alarms, etc. may be sent from the remote server to the MMD. Such embodiments can (but do not necessarily) eliminate the functions of the smartphone. Alternatively, the smartphone may be the hub that sends out the 5G communication (e.g., MMD communicates with smartphone which then forwards messages over the 5G network). The smartphone may still function as an interface with the MMD and interface with external databases and programs (e.g., APIs), displaying results, animations, statistics, etc. in user friendly formats.

Such communications in an ultra-low-latency 5G environment may form a tactile Internet experience. As discussed elsewhere herein, sounds of changing pitch can help guide the user to adjust motion toward the desired motion. Such guidance may be improved in a more fully tactile environment with things such as electrical stimuli (e.g., energizing electrodes on the MMD in contact with skin), spinning up of small physical gyros (e.g., an additional gyro pack inside the MMD, or a separate gyro pack/armband), and/or other devices that may also be energized in a way that guides, effects, or otherwise suggests, helps, or changes a user's motion toward the desired motion. Such other devices may include, for example, loT devices attached to the user or near the user. The loT devices may be publicly owned or accessible loT devices. In one embodiment, additional sounds are provided to the user through remotely connected loT speakers which are energized to provide moving or spatialized sound, such as Barco Auro, DTS:X, or other sound technologies such as Dolby Surround.

In one embodiment, the present invention is an MMD in an ultra-low-latency environment (e.g., 5G) forming a tactile Internet experience, wherein gryo readings from the MMD are transmitted via an ultra-low-latency network to a remote server for analysis comparing those readings to motions for identification and evaluation of the motion. The remote server configured to return signals identifying various analysis that may be, for example, a reward tone indicating a good motion, buzzers, sounds or other haptics identifying motions out of range of the desired motion - and such sounds/haptics may be arranged so as to help guide the user toward the desired motion. In one embodiment, the present invention comprises an MMD that only interacts with a remote server via a network, and a user's smart phone or other device (e.g., computer/website) communicate with the user to pass information from the user to the remote server and receive/display information about performance etc. from the remote server.

The communications to and from the MMD, remote server, smartphone, etc. may be compliant or compatible with one or more standards. Devices such as loT, services, User Equipment (UE), and others in communication with the MMD, the remote server, or smartphone may similarly be compatible. Such devices may include loT, Virtual Reality (VR), control, ubiquitous on-demand coverage, and such devices or variations thereof may be flexible to meet customized data gathering or feedback needs. Such devices include drivers that work within one or more standards including motion, video, navigation, and sound. In various embodiments, the focus being to use the 5G system in real-time (or near real-time) to support use of the MMD via a scalable and customizable network, with appropriate Key Performance Indicators (KPIs) (e.g., latency, reliability, data rates, etc.) at levels that enhance the user experience.

The present invention includes maintaining data as to user motions and classifying different users into group or levels of compliance with various motions (e.g., ranking tennis players based on how closely their backhands match typical professional backhand motions, ranking players of any sport based on consistency of matching professional players in any one or more specific or categories of motions).

The present invention also includes maintaining data about user reactions to various alarms, haptics, or other feedback provided to users and the overall performance improvement of each user in relation to the types of feedback received. The present invention includes updating training regime and/or feedback according to results among all users through the identification of feedback regime that are more effective, and those that are more effective for a particular user.

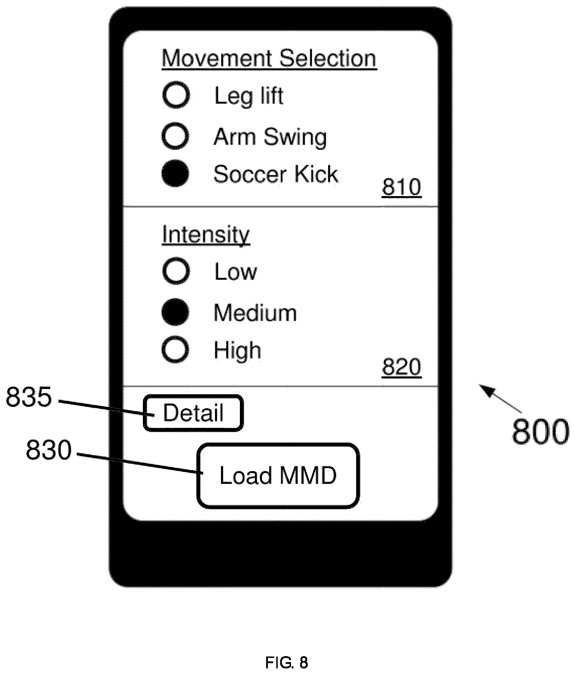

Fig. 8 is a screen shot of an application 800 according to an embodiment of the present invention. Radio buttons 810 are programmed for movement selection, here illustrating a lift, a swing, and a kick. The selections may include any number of other options including programs such as the fitness track program discussed above, a circuit of a gym, recorded moves (which may include options to customize the name), downloaded moves, etc. The programs may include variations of a program, such as program 1, variation A, B, and C, giving the user the same routine with slightly different motions so as to avoid repetitive motion injuries and to maintain additional flexibility.

Radio buttons 820 provide an additional variation as to intensity. Other features may be selected via radio buttons or other user interface types. The application 800 may further provide access (detail 835) to a timeline, chart, or other visual (including video) of a stored motion or combination of a timeline and visual, along with a user interface that allows selection of one or more portions of the motion to which the analysis, comparisons and feedbacks described herein may be applied. Various other preferences, such as volume, tolerances, and/or setting a tone to be played when no evaluation is made may be set, for example. Button 830, then initiates loading of the selected motion or portion thereof.

The MMD may applied to various activities, including, for example, automatic sports tracking at a gym for form at various weight stations, logging workout(s), and double as a wristband that merely id's the individual, and the IMU and other electronics may be automatically programmed to that a user's preferred settings when they are at a workout station. The MMD may also be programmed to provide alerts to a trainer, gym manager, or others if things look bad (e.g., a move does not go as planned, or detects difficult situations, falls, etc.), like dropped weights.

One aspect of the present invention includes recognizing initial patterns of user movement indicative of the stroke to be performed, comparing the performed stroke in real-time or near real time to one or more stored strokes. Such initial patterns may be determined from analysis of the user's data and/or a large data set collected across multiple users (e.g., big data analysis). The present invention includes creating a stroke envelope composed of the limits of several different performances of a same stroke (by a same individual or by a plurality of any of trainers, professionals, groups, therapists, ranges of a big data analysis, etc.), and comparing a user's stroke to the stroke envelope. Such analysis may include dividing each motion into a plurality of segments and applying an envelope to each segment.

In one embodiment, movements of trainers, professionals etc., are fit to a curve to which accelerometer and position data is applied. Accelerometer reading more than a certain degree, percentage, and or predetermined amount from the curve triggers the alert/alarm. The alarm volume may vary based on an amount outside the curve. For example, intensify for (larger amounts) or be subdued (smaller amounts). In another example, the alarm may have a tone frequency or an on/off staccato frequency that varies based on the amount outside the curve. The alarm may include a distinctive cut-off/turn-off sound when it is silenced upon return (or within tolerance) of the curve. Such decisions and comparisons are made, for example, by the electronics of the MMD and transmitted to a smartphone or other device for storage, display, and/or further analysis. Accordingly, the present invention includes a varying alarm wherein the variations are indicative of a characteristic of the motion performed.

Although the present invention may be implemented in various ways as will be understood by the skilled artisan in light of the disclosure herein, the present inventor has found great efficiency with an architecture that provides motion analysis at the MMD and then communicates motion analysis and/or other data to a remote mechanism such as a computing device, smartphone, or other device for either further analysis or display (such as any one or more of data, statistics, and/or simulations).

Motions may be detected in various ways. For example, Table 1 provides exemplary test code for determining a backhand when the MMD is used as a table tennis coach:

**Table 1**

| |
|---|
| ```
     if ((imu.calcGyro(imu.gz)) > 1) {used to be 1
          delay (200);
``` |
| ```
          imu.readGyro();
          if ((imu.calcGyro(imu.gx)) < -5 ) { // used to be -3
              delay (200);
              imu.readGyro();
              if ((imu.calcGyro(imu.gz)) > 3) { // used to be 3
                   delay (200);
                   Serial.println("BACKHAND");
                   imu.readGyro();
                   //testbackhand
               }
              }
``` |

Table 1 illustrates a test case for a user performing a predetermined backhand. The table illustrates a test case where the user initiates a move and the values from the accelerometer (accelerometers) are tested to see if a successful backhand has been performed. As shown in Table 1, values from the gyroscope (or multiple gyroscopes, different gyroscopes set on different axes, for example) are tested to see if they correlate with predetermined values of a backhand.

In one embodiment, multiple gyroscope values are similarly tested in simultaneous or near simultaneous real-time and evaluated against a plurality of moves which may include backhand, forehand, and any number of variations of those moves (e.g., upper-cut backhand topspin, down-cut backhand backspin, x-cut backhand off-table spin, cross-court backhand flat, as a small set of possible moves evaluations). In one embodiment, the evaluations can share comparison values to the extent practical or they may be implemented with separate comparison values for each instance of a move. For each instance of move, stored values may include a range of acceptable values as maximum or minimums, percentages etc.

In one embodiment a percentage is implemented and the percentage varies from one instance to the next such that, for example, in portions of a move where higher accuracy or precision is either necessary or desired lower percentage variance is provided for corresponding instances in the comparison. For example, follow though may have more variance than instances before contact.

In one embodiment, the variances decrease (lower percentage tolerance) with each instance prior to contact and decrease rapidly after contact, such the variances may, for example, continually change but change more rapidly at key points such as just prior and just after contact. The variances (or tolerances) may change from value to value or they may be assigned for a segment, and segments may vary in length or amount/number of data points in each segment.

In this example, the delay(200) is a designated delay in milliseconds between recording/testing values (which is performed 5 times per second) during a specific move. Since table 1 is a test, testing/checking was not necessary at a great speed, which is easily modified to test many times per second such that a continuum of values replicating the move without any one value being significantly higher or lower than its neighboring values even though the direction and/or acceleration through the move changes rapidly when a user observes a move being executed. Such testing may be performed at 300 or more times per second.

An additional example is provided in Table 2.

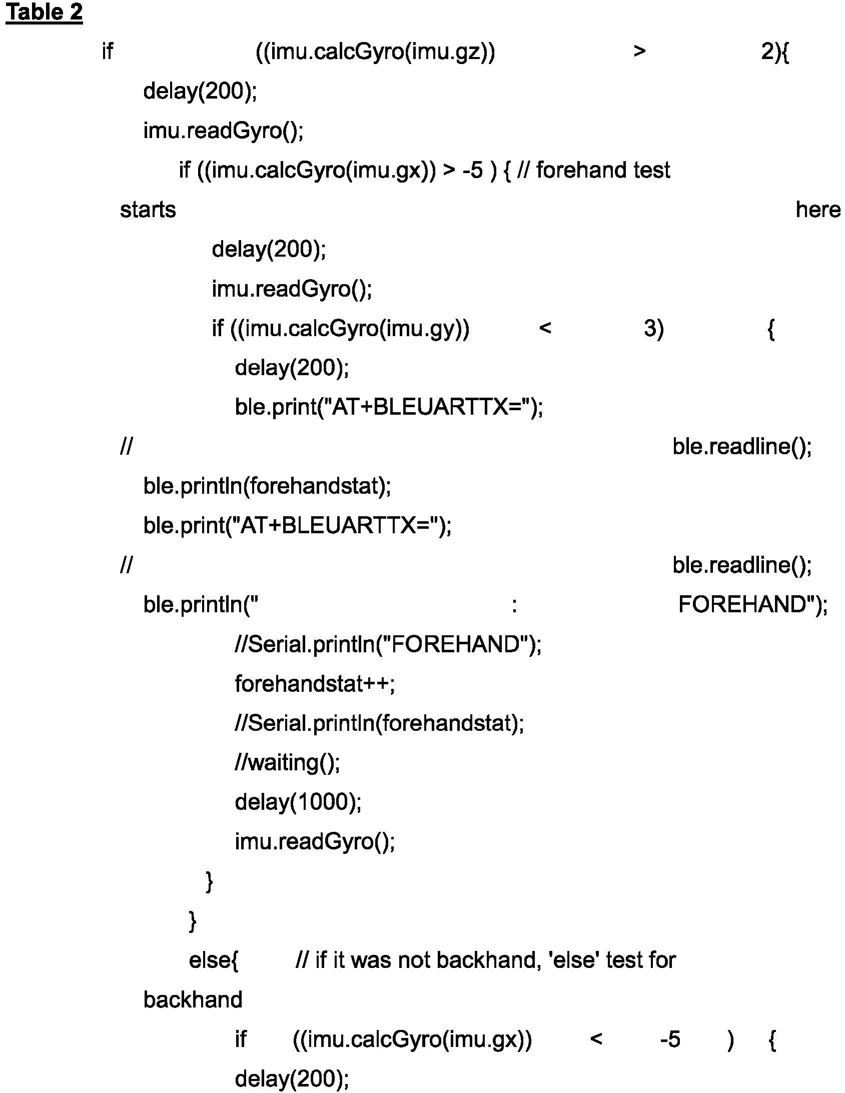

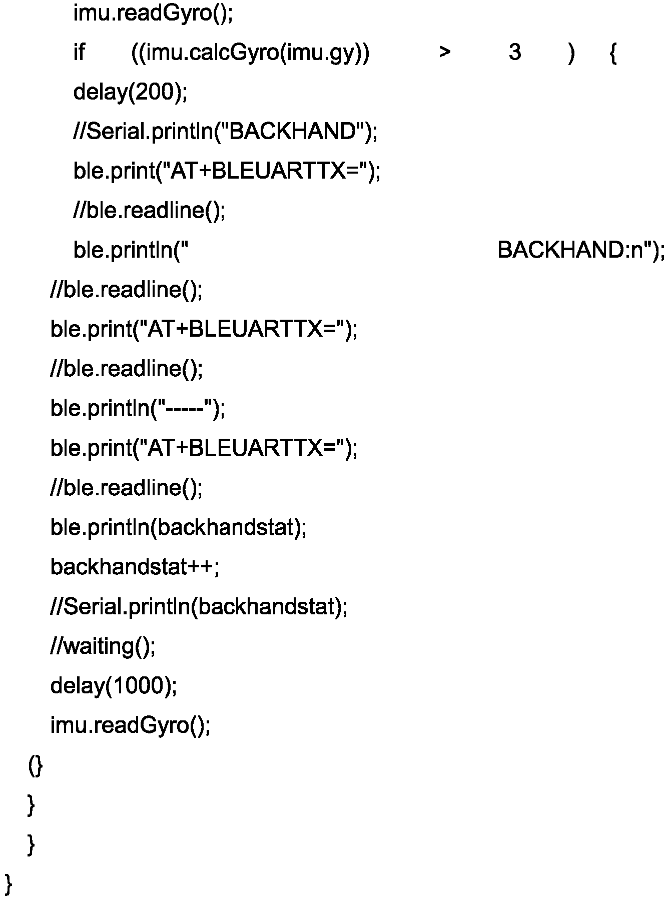

As shown in Table 2, the routine tests first for forehand and then for backhand. The forehand test reads the IMU data for one or more axes. Here, for example, the x-axis of the IMU is read first and tested for a value that indicates a right to left forward swing (e.g., > -5). If the value read is what is expected of a forehand in that axis, then another axis is tested, in this example, the y-axis where a < -3 value (in combination with appropriate x-axis value) affirmatively identifies a forehand.

The present invention includes testing multiple axes for first indications of a particular stroke. The multiple axes may be the x and y axes as provided in Table 2, or it may be a series of different x, y, and/or z axes set in different spatial orientations. The initial motions (and the full motions to evaluate the entire stroke against) for each stroke is preferably provided by the user, coach, or downloaded as discussed herein. The initial motions may be extracted from practice motions by the user or coach. In one embodiment the present invention utilizes a limited number of axes to recognize a motion and then processes a larger number (a plurality) of differently oriented axes to evaluate that particular motion.

If the forehand test fails, the program drops down to test for backhand. The values tested may include the same values previously read from the IMU(s) or they may be freshly read. In at least one embodiment some of the IMU values are shared between tests and some IMU readings are new.

The example in Table 2 is an initial test for particular strokes (forehand, and backhand). The test may be extended to the various other strokes and to sub-categories of strokes (e.g., forehand with top-spin, forehand with backspin, etc.). In one embodiment, the if then else structure is extended for any number of motions. In another embodiment, a programmable test loop repeats and reads in a different set of parameters identifying each of different motions which are tested individually and moving on to the next if the test is not passed. In one embodiment, the first test is the most used motion, thereby eliminating unnecessary tests if the most used motion is tested first. In one embodiment, the MMD tracks motions practiced or used by a user and readjusts programming such that the most common motions are tested first.

Further additional routines are implemented to evaluate the particular stroke and provide feedback in real time as the stroke is practiced. The delay time illustrated (200) is for test purposes and a functional device would test at a much faster pace (e.g., 5ms, 1ms, or less depending on hardware capabilities). Such additional routines may be programmed in a similar architecture such IMU values are read at, for example, predetermined intervals and/or at portions of a motion which are compared to a range of expected values for that motion at that time and which are, for example, provided from a recorded practice move, downloaded, etc.

It should be noted that the ble.println commands implement Bluetooth communication between the MMD and a computing device such as a smartphone app. Here, the command identifies the motion (stroke) as either forehand or backhand. However, in an operation device, such a command may be utilized to signal auditory or vibrations feedback for example. The results will also be sent to a smartphone (or other computing device) which can then utilize the results to implement any of the features discussed herein and may include the ability to review moves including simulated motion, highlighting desirable corrections, etc. The smartphone may also communicate the data for further analysis or storage retrievable by others for yet further comparison, analysis or improvement in either.

The examples provide for communication between the MMD and smartphone as necessary or desired for various smartphone (or other external computing device) features. And all data, which may include raw, MMD pre-processed, MMD processed data, or a combination thereof may be transmitted to enable various external features (such as a smartphone display).

In one embodiment, the MMD does not utilize a smartphone at all and the user interface is clean. The MMD is, for example, programmed to recognize a set of moves, each of which is evaluated and feedback provided to the user directly from the MMD. The feedback is, for example, when a larger than expected value for the identified motion occurs (e.g., too fast) a high pitched or high frequency beep occurs, and, if too slow, a lower pitch or lower frequency beep is sent. The feedback may be implements in an if/then/else programming structure.

In addition to the MMD, the present invention includes collection, consolidation, and analysis of data from disparate devices connected over a 5G network. Such data may include video from cameras positioned to observe practice, play, therapy, or other uses of the MMD. Such data may be collected via cell phone cameras of bystanders, therapists, and others, security cameras. Such data is not limited to video and may include audio, social media posts, data from IoT devices, etc. In some cases, the speed of collection and transmission is sufficient to provide near-real time additions to motion or other analysis performed. Such data may be provided to enhance application experience such as for example, a replay of a motion where the user watches the replay on an app or computer monitor that may include actual video and feedback similar to that provided while performing the motion.

Such replays would typically be performed on a smartphone or computer and include a split screen playback where one side is a video of the overall move (e.g., user's upper body, or entire body) and the other side providing a closeup of the body part whose motion most influences the quality of the move (e.g., hand or wrist). The MMD may also be shown in the videos, and may, for example, light-up, highlight, or other indicia at times when the MMD was buzzing during the move. In one embodiment, the MMD also buzzes on the user's wrist during playback so the user can be more aware of the critical points in the move being reviewed and the motion to be corrected.

The present invention includes use of loT data from different devices, and that data may be amalgamated into databases or other storage in the cloud or at the user's smartphone, computer, etc. Such data may be processed, correlated, and cross-correlated and fed into an API to find patterns or situations that effect a user's workout, therapy, or other activity in either positive or negative ways. Such data is useful to the user in planning for events or other activities where the motion is to be used.

Although the present invention has been described herein with reference to a motherboard IMU circuit board implementation, the devices and processes of the present invention may be applied to other devices including existing platforms having sufficient motion detection and processing power, such as for example, a smart phone may be configured as an MMD, an Apple watch may be configured as an MMD, if running an appropriate program (e.g., application) to capture and analyze motions according to the teachings of the present invention. Further the MMD may take the form of a smartwatch or other device.

In one embodiment, MMDs are utilized on different pieces of equipment, such as on a wrist band and/or on a shoe. The audio prompts may be transmitted to a speaker or an earpiece (e.g., a wireless Invisible In Canal (IIC) device) to guide and/or provide feedback to the user. Collected data may be transmitted to the user's smartphone and the disparate data streams combined in side-by-side or over/under (charts or statistics, for example, e.g., number/percentage of correct moves, degree of variation, average amount outside envelope, etc.).

In one embodiment, MMDs are utilized in conjunction with each member of a team, a subset of a team, and/or members on opposing teams. For example, MMDs installed on shoes of soccer players. Each MMD communicating with a central collection point in communication with a smart phone or other computing device. The collection may be performed by, for example, a network of receivers at different locations on a sideline(s) of a playing field, receivers placed on goalposts, mobile devices on field (e.g., clipped to a referee's belt). The data can be analyzed and provided in graphic form to a coach for evaluation. The data may be displayed to show or identify coordination between the players and providing data for coaching evaluation. Such data may be provided in tandem with video showing the moves corresponding to the data.

In describing preferred embodiments of the present invention illustrated in the drawings, specific terminology is employed for the sake of clarity. However, the present invention is not intended to be limited to the specific terminology so selected, and it is to be understood that each specific element includes all technical equivalents which operate in a similar manner. For example, when describing an accelerometer, compass, or other components, any equivalent or other device having a similar function or capability, whether or not listed herein, may be substituted therewith. Furthermore, the inventor recognizes that newly developed technologies not now known may also be substituted for the described parts and still not depart from the scope of the present invention. All other described items, including, but not limited to smartphones, motherboards, sports equipment, attachment mechanisms, training routines, moves, etc., should also be considered in light of any and all available equivalents.

The present invention includes a device comprising an accelerometer, a processing device configured to receive data from the accelerometer and compare motion(s) indicated by the accelerometer data to previously stored motion(s), and a feedback mechanism configured to notify a user of the device about at least one aspect of a user motion. The device may further comprise a wireless link between the accelerometer and processing device for communicating data from the accelerometer and feedback signals to the feedback mechanism.

The accelerometer and feedback mechanism may be mounted on a wrist/ankle attachable band, and/or the accelerometer may be attached to a sports equipment item. The accelerometer may be embedded in a smart watch.

The feedback mechanism may comprise multiple points of feedback individually energized in a manner representative of motion error identified in the accelerometer data. The feedback may be different for different variations of a desired motion.

The device may calculate at least one aspect of a user motion comprising an amount of deviation from a desired motion. The variation may be communicated to the user via at least one of volume and frequency of the feedback. The amount of variation may comprise an amount of variation in at least one of position at a predetermined portion of the motion and speed at the predetermined portion of the motion. The reference motion may, for example, comprise at least one of a motion recorded by the device via a user motion, a motion recorded by a similar device by a coach or trainer, and a motion pre-recorded and downloaded to the device.

The present invention may be embodied as a method of analyzing motion data, comprising the steps of receiving accelerometer data, identifying a relevant motion in the accelerometer data, comparing the relevant motion to a reference motion, and sending feedback indicative of the comparison. The step of comparing may comprise comparing at least one of up/down (e.g., x axis), left/right (e.g., y axis), and forward/backward (e.g., z axis) motions to the reference motion. (the axes may be labeled differently). The step of comparing may comprise feeding the accelerometer data into an equation representative of the reference motion. The equation may include a margin of acceptable motion error which identifies whether negative feedback signals are to be issued.

The step of comparing comprises comparing data points of accelerometer data to corresponding synched accelerometer datapoints accelerometer of the reference motion. The feedback may comprise an audible or vibrational notification indicative of an incorrect motion. The feedback may comprise a higher pitch audible alarm when the motion is generally too high and a lower pitch when the motion is too low.

The present inventor has further realized that volume and other parameters of the feedback may be altered or changed while the feedback is being provided and that the provided feedback is simultaneous with the motion being evaluated. In one example, such feedback may have the volume of the feedback altered based on an amount of deviation of the motion being performed and a stored motion being replicated. For example, a user makes a motion which starts out basically correct deviates more significantly, but finishes with a nice follow through that matches the follow through of the stored motion - in this case, the volume of the feedback would start out low rapidly increase through the motion and tail off (and actually stop) as the user follows through with the motion. In this manner the main part of the feedback occurs entirely while the user is in motion and feedback ceases before the motion is complete because the motion finished correctly (or within a predetermined margin of allowable motion deviation).

The method may further comprise the step of communication with a remote device operated by a professional and receiving or downloading prescribed or suggested moves from the remote device. And, the method may further comprise a step of recording accelerometer data during a demonstration motion and using the recorded accelerometer data to determine if a subsequent motion is similar within a predetermined envelope. In one example, the remote device may be an automated machine designed to pitch balls, serve, or perform any of the functions of automated machines (e.g., ping pong robots, pitching machines, etc.). In the case of a ping pong robot, for example, the robot may be programmed or provided a series of shots specifically designed to test the motion the user has loaded into his/her device. The robot (or robot controlling device) may receive communications about how well the user is performing and may up the speed, spin, or other characteristics of the served ball, or may further vary such parameters around weak spots, that may be based on the amount or type of feedback the user is receiving.

The present inventor has realized that improved techniques and methods are needed to efficiently capture and accurately evaluate motions and other processes herein which may be summarily described in some embodiments as a digital coaching device. Processing speed and accuracy is an area that may be improved by focusing somewhat on describing and capturing the motions as timestamps - for example, only timestamps. Such timestamps may be recorded by the IMU during a certain time period. Such time period may be an amount of time after a motion has started which may be identified in a number of ways, including, for example, comparing a series of time stamps that show a similar path (such as the start of a motion) to a prerecorded motion which itself may be timestamps. And in such case, the IMU records and keeps track of all the information it is able to record such as velocity, acceleration, position, rotation, pitch, yaw and all data points it collects over time. Over time means, for example, that it is able to record all these values several hundred times per second.

The device is arranged (via programing or electronics) to use these data points and the whole data structure (recorded by the IMU) to do calculations and feedback that is unique to the present invention, the situations, and goals of the present invention.

In various embodiments, by pressing the button of the device, the user is able to record (and also stop with another pressing of the button, this can also be done on the 3rd party device such as phone) the timestamp and data structure that the IMU is collecting until the button is pressed again to turn the recording off. For example, repeatedly timestamping each motion, acceleration, and or position information produced by the IMU in rapid succession and storing the same in the data structure. This is the recording function the device and recording of motion/path which may be, for example, a collection of data points in time. The data structure may be passed as an argument in a subroutine call or as a parameter in an API or directly device to device (such as from the device to a table tennis robot or other computing device (e.g., smartphone) for further analysis, prediction, etc.). This type of structure allows for efficient management and use of the motion data and parameters.

The data structure may further include one or more reference points, for example, when the user selects a portion of a motion to practice, the data structure may contain one or more timestamps noted as a starting position and/or a stopping position that highlight the portion of the motion to be practiced or drilled. The data structure may include weighted points as well identifying sections of the motion that are to be weighted more strictly in any analysis. For example, a motion may include 3 or more portions, such as a set-up portion, an impact portion, and a follow-through portion, each of which may be weighted differently. The weighting may be, for example, an allowable tolerance or margin of error, such as a percentage variation from the recorded parameters. For example, in one embodiment, a set-up portion may have a 20% allowable variation, an impact portion 5% allowable variation, and a follow-through 30% allowable variation. The variation may be set as a combination of speed and position differences, each of which are typically evaluated separately and have their own type of feedback associated (e.g., different parameters, different feedback types), or, alternatively, the parameters may be combined and evaluated at the same time and same feedback (varying by amount of variance from the reference motion, for example).

The various portions of the move may be segmented into parts automatically or the user may engage an app or other program as also described elsewhere herein that allows the user to segment the motion into the parts s/he wishes to work on. Such app may also allow percentages or other indicia of tolerance to be associated with each segment or portion of the motion. The app my further allow the user to select other parameters of variables and set values that alter any of the comparison or the type or amount of feedback provided (e.g., selecting or setting of prediction settings for each segment or the motion as a whole, feedback during the motion, and/or post motion coaching).

The device is of course capable of real time feedback based on the comparison of the moves, but further now the device uses a more efficient method of updating how the feedback is actually given. When the user presses the button (which may be one press or long press, or pattern of presses, e.g., two or three presses for example), holding it pressed for a second or so to go to training mode, then the device compares the currently performed movement to the previously recorded movement. However the change is in the feedback. The feedback can and also doesn't have to be given during the middle of the execution of the move. Such as in the beginning for instance the user may be off the original path and so the device is capable of giving multiple forms of feedback simultaneously such as sounds, audio commands and vibration (which may together indicate a motion variance, or motion variances of multiple different aspects of the motion). However if the user would during the later portion of the move be on the correct path (e.g., within a specified tolerance) of the original movement, the feedback would stop, thus telling the user that part of the movement is correct.

Also the functions of the feedback can be 'reversed' where the user still gets certain feedback even if they are correct when doing the practice move (perhaps very light audio sound ensuring them they are correct) in order to really let them know (without just being silent) that they are correct. Such as positive reinforcement, to create the positive energy of a real coach.

A move/motion (or datapoints) that are recorded in a move constitute a whole recording that was done with the device. However only a certain part of that move/motion is actually relevant. Going into the proper stance, preparation and follow through are not always part of the movement the user intends to practice. The user has the option and ability (on the phone) to 'trim' the movement to include only the relevant part they want to practice. However, based on the preparation move and the follow through we can give feedback to the user to 'get into the proper stance' so they start their movement correctly. This can be in all forms of feedback, but the best way would be an audio feedback instructing the user how to get into the proper stance and preparation and when to actually start the ideal move they want to practice.

While the "irrelevant" motions may be trimmed and/or not utilized in evaluating the motion at issue, those irrelevant motions can provide information, especially when evaluated over time, that may improve accuracy and prediction of the motion at issue. Preliminarily, the set-up, stance, etc. may itself need improvement and can be a "motion" evaluated directly. Further, even if the setup and stance, etc., are not evaluated directly, recording information about those motions or positions can be evaluated and utilized for other purposes or to provide preliminary information about the users preparedness or statistics/coaching feedback. For example, by evaluating such motions in the background and comparing them to successes and failures in training or during matches, the device can gain knowledge (e.g., like Poker tells) about the user. For example, it may be determined that if the user begins to lean left in his stance that he is unlikely to complete certain motions properly such as a reverse spin return. Such tells may be fed back to a robot or other device practicing with the user, and the user may also be made aware of the "tells" so they can potentially be eliminated or utilized to the user's advantage. Various embodiments may include using tells in deciding automated serve characteristics or other automatic practice devices. Feedback from the device may be utilized to illustrate the tell such as a short burst of pulses warning the user as he leans left or right in his/her stance (e.g. pre-feedback). The robot may be programmed to recognize this and serve more right when the user is actively leaning left (or as the user initiates his lean), for example. The user may be informed about such practices before or after they are utilized.

In one embodiment, the MMD device performs all the analysis and packages an abbreviated instruction set to the robot which may include, for example, change parameters for the previous shot, such as, for example, "+10 L S", meaning 10% more left spin. Such instruction may arrive after a shot and/or anytime before a next shot or before a next similar shot (e.g., in the case where the robot changes each shot, the change parameters would be additive to the next similar shot - and, of course, this is just an example, the change parameters may also be applied to dissimilar shots or only to the exact same shot.)

In one embodiment, a tell is recognized and change parameters are sent to the robot based on the tell and to be applied to the next shot which is about to happen. Change parameters based on the tell may be to take advantage of the tell, such as helping to illustrate to the user the disadvantage the tell causes (change parameters making it more difficult to return the shot), and/or to give the user the advantage of the tell action (change parameters making it easier for the user to return the shot). The change parameters and the return shot are, for example, coupled with feedback from the device during the shot that may be amplified (further from the recorded motion) and subdued (closer to the recorded motion) during the shot to illustrate how effectively the user is following a recorded motion.

In one embodiment, the following steps may be taken:
- 00100 Recognizing, by the MMD device, a tell based on, for example, a user's stance, ready position, motions before, during, or after the ready position, predictive analysis;
- 00200 Preparing a change message noting information about the tell and/or a change in shot because of the tell;
- 0300 Sending the change message to a robot or automated machine;
   - 00400 At the robot or machine, changing any of a placement, speed, angle, force, vibration, video play, coaching dialog, spin, or other parameter of a shot, feedback, or other function of/from the machine;
- 00500 Recognizing, in real-time, differences in a user's motion compared to a recorded or planned motion;
- 00600 Providing feedback to the user while the motion is occurring (e.g., providing feedback that varies while the motion is occurring indicative of how far the user is from the recorded or planned motion, the indicative feedback varying while the motion is occurring and is based on, for example, a predictive analysis based on the tell and past user motion's such that the device "knows" how the user will perform the motion).

The tell may be a combination of user actions, stance, etc., and physiological feedback such as eye-movement, heart rate, blood pressure, etc. Physiological data may be provided by sensors built into the device or provided by separate and/or stand alone devices.

In one embodiment, the machine is a Peloton-like (live or prerecorded) on-line coaching workout device. The tell or feedback may be, for example, a foot's angular position on a pedal, grip on handlebars, and/or may be further combined with existing Peloton-like data, such as Cadence or spin-speed.

In one embodiment, a power equation may be utilized in recognizing the tell and/or formulating the machine's response. Further, in some embodiments, such as, for example, when using an interactive, AI, or static based coaching, the change parameters may alert the "coach" to a tell who may then indicate (keep weight off the left leg, straighten your ankle, your leaning left, etc.).

In one embodiment, a device that recognizes a tell and alerts another program (such as a coaching program) to the tell where it is used to provide coaching assistance to either correct an error or enhance a good action of the user. In a further embodiment, modifying a coaching response based on a tell or informing an actual coach about the tell while the user is performing (such as riding a spin-bike, playing table tennis, etc.). For example, in a program with one or more typical coaching responses, changing the typical response to one modified according to the tell. In one embodiment, the MMD device initiates a combination of 2 or more (or all) of a coaching response, a shot (or other machine action) change, and feedback that varies in any of intensity, pitch, vibration corresponding to an amount of deviation from a stored motion during a motion by the user.

In one embodiment, the tell is not recognized by the device, but instead, the device provides motion data to, for example, a connected Al analysis or machine learning environment which derives the tell from the motion data and may then transmit any recognized tells (or any predictive analysis related to analyzing the user's subsequent motion) to the robot or other device.

Accordingly, various embodiments may utilize an analysis of the user's performance as determined by the comparison of motion to a saved motion over time (e.g., a plurality of practice moves) and feed that information to other devices such as a ball machine, treadmill, cycle (e.g., Peloton), rowing machine, or other devices to modify any one or more parameters of the device(s). For example, in a cycling workout, the comparisons may reveal the user is having problems maintaining form and the data may be utilized to keep the user on flat terrain with coaching (which, in this case, may be a Peloton-like coach speaking to the user through a display terminal) as opposed to additional hill climbs. The same type of decisions may be made for any of the workout devices for which programming may be implemented to address any problem identified through the motion comparisons and may be coupled with outside coaching or explanation of what the user needs to work on. For example, a wearable device configured to capture a user's motion and transmit data of one of the captured motion, information about the captured motion, a comparison of the motion to a stored motion, and/or information about the comparison to a remote device interacting with the user wherein the transmitted data changes a parameter such as speed, direction, or other qualities of the remote device. In one alternative, the information is transmitted to a remote smart coach or a human coach that transmits coaching instructions related to the compared motions over an Internet or video feed to the user.

In case the user hasn't used the device for some time and now suddenly decides to practice again, their history of movements is recorded already. Such history may be stored on the device or remotely (in the cloud, for example). Based on that history, the device can calculate how they moved in the past and what the most likely scenario will be of them moving now (their most likely path or movement). Such predictions may be based on the most relevant portions of a motion, the entire motion, and/or other parts such as the stance, set-up, etc., to include, for example, any combination of the above. Based on that, give instructive feedback based on their previous mistakes and misalignments. This can not only be instructing the user with commands such as "left, right, faster..' but can also be a quick summary of what their previous tendencies were, what to watch out for and how to correct them properly. This can be both in audio form and also on smartphone display, without being long and boring. For example, it may go straight to the point with few keywords analyzing their move history - such as, any one or more of known tendencies which may include too slow, tilted to the left, not swinging high enough; improve speed, left positioning... etc.

Another added feedback is an overall feedback after the completion of a move. Currently the users are getting real time feedback during the practice, but the third (first and second were audio and vibration feedbacks in real time) feedback is the overall feedback that the user gets after a practice execution of the move. Here, for example, the whole movement that the user performed may be summarized. Not just simple sounds and vibration, but an overall summary - both audio and visual/text description on their phone. This is to give the added bonus of a real (or more real-like virtual) coach that helps their students with correcting their forms in real life and giving them feedback based on their performance and executions. In one embodiment, a comprehensive coaching feedback is provided. The comprehensive coaching feedback may take the form of commentary about the move (e.g., a voice stating, for example, "You started the move high but you finished correctly.") In one embodiment, the coaching feedback includes both what the user did wrong and what the user did correctly thereby providing at least some positive feedback along with corrections. The positive feedback may come with every coaching feedback or be randomly included, so as a reinforcement it is more effective. The positive feedback may be provided when the user starts to do things right but less frequently (or not at all) or in an abbreviated form when the device recognizes that the user has fully mastered the move. Accordingly, various embodiments may include collecting data of the user's proficiency of certain or all identifiable moves such that appropriate positive feedback may be provided and not inundating the user with too much or unnecessary feedback.

In one embodiment, the user's proficiency of various moves may be linked or otherwise communicated with an on-line gaming platform such as Roblox (or a stand alone game) and used, for example, in unlocking levels, weapons, or other items. A similar paradigm may be provided to other platforms based on other activities. For example, a cross country coach may provide devices to his/her team and any of mileage, course times, etc., tracked by the device may be used to unlock levels in a corresponding cross-country gaming environment or any other gaming environment level, weapons, or other items (e.g., cross- country skills or stamina equating to extra ammo, better weapons, or levels in a shooting game). That last point in the parenthetical proposing a transfer of skills or work ethic in the physical world to points or progress in a virtual world that may not be related to the real world activity that is tracked and monitored (although some synergy is expected when the real world activity and the virtual world have some common nexus such as real world soccer practice and virtual soccer in a gaming environment). Accordingly, in one embodiment, tracking of a user's movements and recording improvements and progress (e.g., compared to stored movements) in the user's goals may be applied to gain rewards for such progress in a gaming environment related or unrelated to the motion. This can be used to motivate the user to perfect his/her moves so that gaming progress may also be realized.

The positive feedback may, for example, be turned off in the settings if the user prefers a shorter coaching session limited to saying what went wrong. In one embodiment, positive feedback and other feedback provided may be tracked and stored in the data structure associated with a motion. Such feedbacks may be utilized in predictive or reporting and transmitted with the move to other devices programs or applications. In one embodiment, the feedbacks are sent to a platform that publishes the feedback and/or various other data on a social media or club platform/application or automatically feeds the caricature into a signature line or header of an email, for example. In one embodiment, the platform publishes a caricature (e.g., of the user) performing the move using parameters or other data contained in the data structure, and such caricature may be a moving GIF that illustrates the move and may include a highlighting of the portions of the motion that are out of tolerance. Such caricature may be the best, worst, last, a series (e.g., from first to last, or other conglomeration), a summation, or average of recent moves performed by the user, for example.

In various embodiments, a secondary feedback or 'warning' may come into play, really critiquing the user that they are not improving and are continuing to make the same mistakes. Such may be implemented when the user is not improving over a practice or a series of practices, or over a predetermined length of time. The critiquing may be more strict in terms of feedback focusing on the 'worst' part of the move that the user is getting wrong. In one embodiment, the critique may be steadily or exponentially increased. This is to ensure that the user would be inclined to get the overall movement at least to a decent overall correct motion.

Over time, the device may also collect intelligence on the user's moves and point out tells that the user is telegraphing such the tell may be stopped or controlled, and, after recognition and control, may be used by the user to throw-off an opponent in a tournament, for example. In one embodiment, feedback to a remote device (such as a ping pong robot) specifically calls out or provides the tell (which may be in the form of raw or processed data) and the robot is instructed to shoot, spin, or place the ball in a manner that either helps correct the tell or takes advantage of the tell to expose weakness associated with it.

In terms on trajectory feedback on the phone and an overall feedback, looking at a trajectory and comparing it might be challenging. However, various embodiments may include simplifying this feedback where the phone display would basically be a spherical representation of the position of the device, and the feedback is given in terms of G-force feedback (e.g., a g-force diagram that for example drivers experience when driving a car). Sides and edges of the phone would just be lighting up with different colors (from red to green, with different brightness strengths) to simply give feedback based on where the user currently is imperfect and where they are out of bounds compared to the original ideal movement.

The above could be implemented on the device itself, where different sides of the device light up to give the light indication right on the device when the user is wearing it - constituting yet another form of feedback that the MMD can provide, for example hearing impaired users so they get the same benefit of the device. An implementation of the above may be for example, a strip of individually controllable LED (e.g., micro LEDs) in a linear or multidimensional array, and the feedback may be illuminating one or more LEDs, illuminating a pattern of LEDs, and/or implementing a moving object (such as an arrow) along an edge of the device. It is proposed that very small but bright LEDs would be most effective. The LEDs may constitute a display and images such as a representation of the motion may be played with highlights on areas for improvement, for example.

In one embodiment, the above and other variations may be implemented as a directional device that may work, for example, similar to a compass (e.g., showing one direction "north" all the time). Additionally, the device can 'light up' the correct direction in which the user should be moving, thus giving the user a visual representation 'Oh when I move from top to bottom the color is green and navigating me in which direction I should be moving. Such light indication can also act as a tertiary teaching method, completely ignoring the speed of the movement that should be done, but rather instructing on the proper path of the movement the user should be executing. The speed of the practiced move is not of the highest importance, rather the focus is on the proper path of the original movement. The speed of the executed movement can then be increased after the proper path of the movement is learned. For example, the user performs a slow maneuver while watching the device. And the same lighting may occur but faster in real time when the motion is performed in real time. In one embodiment, the device recognizes the slow maneuver based on the motion and slow speed and may, for example, recalibrate the motion such that time stamps of the actual motion are replicated and/or interpolated to prepare a reference motion that is "stretched out" for comparison. In other embodiments, the timestamps are not replicated but the timing of each is increased to match the user's rate of change in the motion which may also change throughout the motion - the device matching the user's timing rather than issuing timing or speed alerts which would be generated in the normal operational mode(s).

Therefore, in various embodiments, the device is actually navigating the user into proper movement technique without being too intrusive with the sounds and vibrations, making it easier on the user where they do not have to focus on difficulties such as speed, quick movements, rotations etc., such as, for example, practicing a move in slow motion. But generally ease them into basic movement before incorporating the more difficult steps of proper techniques.

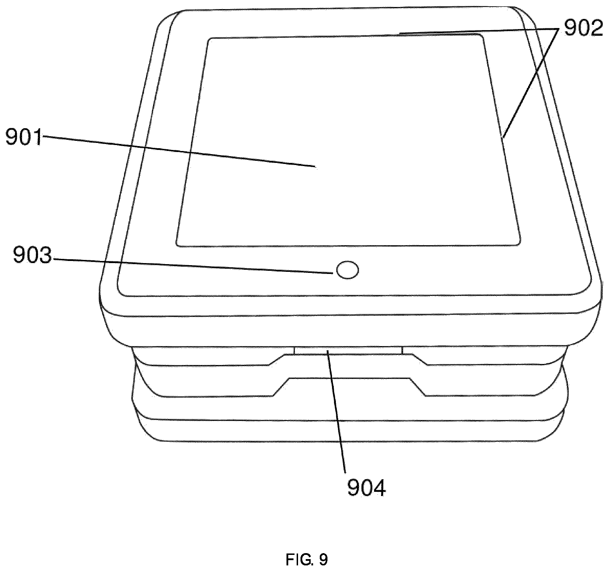

Fig. 9 illustrates a design of an MMD device that may be wrist or ankle worn via a strap or band. The device may also be placed in a specialized pocket on clothing but is preferably secured such that the device is stationary relative to a part of the body for which measurements are taken - but may be placed in standard pockets unsecured as well. The device may be further attached to equipment such as bats, shoes, racquets, gloves (e.g., baseball mitt) etc. The device may be glued into position or use Velcro or screw on attachments, buckles, latches among other attachment methods.

The exterior design of the MMD device includes 901 - Turn on/off button, 902 - Lighting feedback indicators which may operate as described above providing visual feedback, for example, during performance of a motion. The lighting indicators may also be used to confirm start-up, shut down, and recording performed by the device. In one embodiment, the lighting indicators (and/or any of audio and/or vibrational feedback) may be narrowed such that only one part of a motion is provided feedback. This may be useful in the case where the user is only working/drilling on one particular aspect of a stroke or motion. In this case, the recorded motion may be limited to the selected portion of the motion and feedback is provided only on that portion of the motion. This may be implemented via programming on selection of motion parts from a connected application or other means. The device may utilize the ready position and early portions of a motion to recognize the motion being drilled and then provide feedback during the selected part of the motion.

Accordingly, the user may use an app which accesses a plurality of stored motions any one of which may be uploaded to the device. The user may view on the app a timeline of a motion and highlight a section of the motion the user wishes to practice and set the device to only provide feedback for that portion of the motion. In one embodiment, the feedback occurs only during the requested part of the motion and is absent at other times. In another embodiment, a steady tone or other feedback is present until the selected part of the motion during which the feedback varies as described above. In yet another embodiment, a steady tone or other feedback is present until the selected part of the move, then a second tone is presented which remains steady if the user's motion is within an acceptable deviation range from the recorded motion, but varies in accordance with a magnitude of the deviation if outside the acceptable deviation range - in this manner the user is notified as to which part of the motion is being monitored and his/her attention may be focused.

In one embodiment, different segments of the motion (or selected portion of the motion) have varying levels of acceptable deviation. For example, a lead up to contact with a ball in ping-pong may have a higher acceptable deviation and the moment(s) of contact have a second higher level of acceptable deviation, and a follow-through portion of the motion may have yet a third level of acceptable deviation. In such cases, the amount of feedback may be less sensitive in portions of the motion that carry higher levels of acceptable deviation.

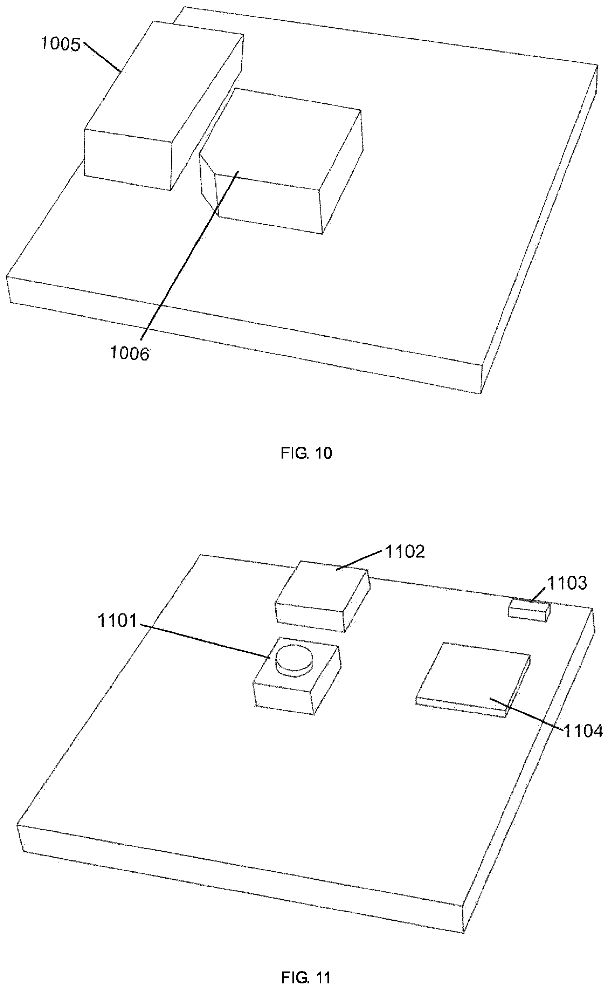

Returning to Fig. 9, 903 is a position alignment mark, and 904 denotes battery charging connectors. In Fig. 10, there is illustrated installed vibrational (vibrational motor 1005) and audio (sound feedback speaker 1006) feedback mechanisms. And in Fig. 11, Turn on/off button 1101, IMU sensor 1102, Board LED 1103, and CPU 1104 also installed on the board. Fig. 12 providing component names and corresponding numerals.

Portions of the various embodiments may be conveniently implemented using a conventional general purpose or a specialized digital computer or microprocessor programmed according to the teachings of the present disclosure, as will be apparent to those skilled in the computer art.

Appropriate software coding can readily be prepared by skilled programmers based on the teachings of the present disclosure, as will be apparent to those skilled in the software art. The invention may be implemented by the preparation of application specific integrated circuits or by interconnecting an appropriate network of conventional component circuits, as will be readily apparent to those skilled in the art based on the present disclosure.

The present invention includes a computer program product which is a storage medium (media) having instructions stored thereon/in which can be used to control, or cause, a computer to perform any of the processes of the present invention. The storage medium can include, but is not limited to, any type of disk including floppy disks, mini disks (MD's), optical discs, DVD, HD-DVD, Blue-ray, CD-ROMS, CD or DVD RW+/-, micro-drive, and magneto-optical disks, ROMs, RAMs, EPROMs, EEPROMs, DRAMs, VRAMs, flash memory devices (including flash cards, memory sticks), magnetic or optical cards, SIM cards, MEMS, nanosystems (including molecular memory ICs), RAID devices, remote data storage/archive/warehousing, or any type of media or device suitable for storing instructions and/or data.

Stored on any one of the computer readable medium (media), the present invention includes software for controlling both the hardware of the general purpose/specialized computer or microprocessor, and for enabling the computer or microprocessor to interact with a human user or other mechanism utilizing the results of the present invention. Such software may include, but is not limited to, device drivers, operating systems, and user applications. Ultimately, such computer readable media further includes software for performing the present invention, as described in the various embodiments and claims.

Included in the programming (software) of the general/specialized computer or microprocessor are software modules for implementing the teachings of the present invention, including, but not limited to, recording motion data, analyzing motion data, comparing motion data, outputting alerts/alarms/sounds, including voices, and the display, storage, or communication of results according to the processes of the present invention.

The present invention may suitably comprise, consist of, or consist essentially of, any of element, part, or feature of the invention and their equivalents as described herein. Further, the present invention illustratively disclosed herein may be practiced in the absence of any element, whether or not specifically disclosed herein. Obviously, numerous modifications and variations of the present invention are possible in light of the above teachings. It is therefore to be understood that within the scope of any claims, the invention may be practiced otherwise than as specifically described herein.

## Claims

1. A device comprising:
an accelerometer;
a processing device configured to receive data from the accelerometer and compare motion(s) indicated by the accelerometer data to previously stored motion(s);
a feedback mechanism configured to notify a user of the device about at least one aspect of a user motion;
wherein the feedback is based on a comparison of a user motion captured in the received data of a practice motion of less than full speed to a previously stored same motion in full speed.

2. The device according to Claim 1, wherein the feedback comprises a visual representation of which direction the user should be moving.

3. The device according to Claim 2, wherein the visual representation comprises lights illuminated in a correct direction the user should be moving.

4. The device according to Claim 2, wherein the visual representation comprises lights having a color representative of a direction the user should be moving.

5. The device according to Claim 2, wherein the user's motion is evaluated without taking account of the motion's speed.

6. A motion device configured to compare a stored motion to a motion being performed by a user wearing the motion device wherein the comparison accounts for speed differences between the stored motion and the motion being performed as if the motions were performed at the same speed.

7. The motion device according to Claim 6, wherein the stored motion comprises an full-speed motion comprising a set of accelerometer-like data points throughout the stored motion, wherein the comparison adjusts one of the stored motion or the motion being performed by one of adding, removing, or interpolating data points therein to compensate for speed such that the motions are capable of being compared at similar points.

8. The motion device according to Claim 7, wherein the adding, removing, or interpolating is performed at different rates through different portions of the motion.

9. A device comprising:
an accelerometer;
a processing device configured to receive data from the accelerometer and compare motion(s) indicated by the accelerometer data to previously stored motion(s); a communication mechanism configured to send an indicia of proficiency of a user motion relative to the previously stored motion(s) to an online gaming platform.

10. The device according to Claim 9, wherein the online gaming platform utilizes the user's proficiency to enable one or more features of one or more games on the platform.

11. The device according to Claim 10, wherein the feature(s) comprise one or more of a level, a weapon, a tool, a key or other items in one or more of the games.
